# EUROPÄISCHE PATENTANMELDUNG

(11) **EP 2 871 233 A1**
(43) Veröffentlichungstag der Anmeldung: **13.05.2015**
(21) Anmeldenummer: 13192606.5
(22) Anmeldetag: 12.11.2013
(51) Int. Cl.: C12N 5/071

(54) **Verfahren zur Herstellung von biogenen Stoffen**

(71) Anmelder: Brandenburgische Technische Universität Cottbus-Senftenberg, 01968 Senftenberg (DE)
(72) Erfinder: Scheibner, Katrin, 01968 Senftenberg (DE); Küpper, Jan-Heiner, 01968 Großkoschen (DE); Schmidtke, Kai-Uwe, 07747 Jena (DE); Miethbauer, Sebastian, 07743 Jena (DE); Herzog, Natalie, 03046 Cottbus (DE)
(74) Vertreter: Simandi, Claus

(57) **Zusammenfassung**

Die vorliegende Erfindung betrifft ein Verfahren zur Herstellung von biogenen Stoffen, wobei a) mindestens ein Ausgangsstoff mit b) mindestens einem Enzym versetzt wird und das erhaltene Produkt aus b) mit c) mindestens einer Leberzelle versetzt wird, und d) mindestens ein biogener Stoff isoliert wird.

## Beschreibung

Gegenstand der Erfindung ist ein Verfahren zur Herstellung von biogenen Stoffen.

Es besteht ein hohes Bedürfnis an verbesserten Herstellungsverfahren für biogene Stoffe. Besonders bevorzugt ist die Herstellung solcher biogenen Stoffe, die bei der Biotransformation oder Metabolisierung in Leberzellen (Hepatozyten) bzw. in der Leber entstehen.

Die Leber ist ein zentrales Entgiftungsorgan des Stoffwechsels. Leberzellen (Hepatozyten) repräsentieren 70-80% aller Zellen in der Leber und verfügen über die wichtgen physiologischen Leberfunktionen (Elaut et al. (2006)).

Die Leber verwendet die Biotransformation oder Metabolisierung um aufgenommene Substanzen (z.B. Medikamente, Toxine, Naturstoffe) ausscheiden zu können bzw. zu entgiften. Für die Biotransformation wichtig sind insbesondere die Phase-I-Enzyme des Cytochrom-P-450 (CYP450) Systems. Es handelt sich bei den CYP450-Enyzmen um Oxidoreduktasen, die einen oxidativen Abbau bzw. Metabolisierung zahlreicher Substanzen wie u.a. auch Arzneistoffe bewirken. Von den zahlreichen CYP450-Isoenzymen mit unterschiedlicher Substratspezifität, die es beim Menschen gibt, sind alleine die Isoenzyme CYP1A2, -2C9, -2C19, -2D6, - 2E1 und -3A4 für ca. 90 % sämtlicher oxidativer Metabolisierungen von Medikamenten verantwortlich (Arimoto (2006); Shimada *et al.* (1994); Lamb *et al.* (2007)). In vielen Fällen erhalten eine Vielzahl von Medikamenten erst durch diese biochemische Veränderungen ihre curative Wirksamkeit oder verursachen gar giftige Metaboliten mit unerwünschten Arzneimittelwirkungen (Chang et al. 2007).

Diese Stoffe sind vor allem als Abbauprodukte der Leber interessant und bedürfen der weiteren Untersuchung. Hierzu sind solche Lebermetaboliten in ausreichnder Menge herzustellen, insbesondere weisen diese biogenen Stoffe eine hohe regioselektive und stereoselektive Spezifität auf. Solche Stereoisomeren weisen regio- als auch stereoselektive Modifkationen auf, die charakteristisch für biotransformatorische Enzyme sind, wie nachfolgend beschrieben.

Für die Biotransformation oder Metabolisierung in Leberzellen sind Phase I Enzyme beschrieben, insbesondere des Cytochrom-P-450 (CYP450) Systems, so genannte Oxidoreduktasen. Weiterhin sind Phase II Emzyme relevant, wie z.B. N-Acetyltransferasen [NATs], UDP-Glucuronosyltransferasen, Sulfotransferasen. Für die Beurteilung der Lebertoxizität von Substanzen ist die Aktivität der Phase-I-Enzyme und Phase-II-Enzyme sowie weiterer Leberfunktionen von entscheidender Bedeutung.

Beachtlich ist weiterhin, dass solche biotransformatorische Enzyme evolutionär bedingt ebenfalls in anderen Organismen, wie Pilze und Bakterien vorkommen können.

WO 2008/119780 A2 beschreibt ein Verfahren zur enzymatischen Hydroxylierung nicht-aktivierter Kohlenwasserstoffe, insbesondere aromatischer Ringe nicht-aktivierter Kohlenwasserstoffmoleküle (beispielsweise die selektive Umsetzung von Naphthalin zu 1-Naphthol) durch Verwendung von pilzlichen Peroxidasen aus Basidiomyceten der Familie Bolbitiaceae (z.B. Agrocybe sp.) zur Herstellung von Pharmazeutika, Terpenen, Steroiden oder Fettsäuren. DE102008034829 A1 offenbart ein einstufiges, enzymatisches Verfahren zur regioselektiven Hydroxylierung von 2-Phenoxypropionsäure zu 2-(4-Hydroxyphenoxy)propionsäure. Die enantioselektive und regioselektive Monohydroxylierung von 2-Phenoxypropionsäure zu 2-(4-Hydroxyphenoxy)propionsäure durch isolierte Biokatalysatoren (in vitro) kann ebenfalls durch ein stabiles extrazelluläres Pilzenzym, die Agrocybe-aegerita-Peroxygenase (AaP), 2-Phenoxypropionsäure hoch regioselektiv zu 2-(4-Hydroxyphenoxy)propionsäure und bevorzugt zu dessen (R)-Enantiomer umgesetzt werden.

Im Stand der Technik ist die Herstellung von biogenen Stoffen mittels Koppelung von Enymen und Leberzellen nicht beschrieben.

Im Stand der Technik ist nachteilig, dass biogene Stoffe nicht in ausreichender Ausbeute und Vielfalt hergestellt werden können. Zudem werden im Stand der Technik zumeist Vorläufermoleküle synthetisiert. Oftmals sind zusätzlich halbsynthetische Verfahren erforderlich, so dass die erforderlichen weiteren regio- und stereoselektiven Modifikationen in die Substanz(en) aufwändig eingeführt werden können, die für Metabolite aus der Leber entscheidend sind.

Die Erfindung betrifft daher die Herstellung von biogenen Stoffen, die nach einem neuen Verfahren hergestellt werden.

Erfindungswesentlich ist, dass die Synthese von biogenen Stoffen unter Einsatz von Enzymen in Kombination mit einem Leberzellsystem erfolgt. In einer bevorzugten Ausführungsform werden die Enzyme ausgewählt aus der Gruppe der Oxidoreduktasen, insbesondere Monooxygenasen, Dioxygenasen, Oxidasen, Dehydrogenasen, Reduktasen und Peroxygenasen. Besonders bevorzugt sind Peroxygenasen. Weiterhin sind Enzyme der Biotransformation geeignet, insbesondere Phase-I-Enzyme und/oder Phase-II-Enzyme. Darüber hinaus sind Esterasen, Hydrolasen und Transferasen geeignet. Entsprechende Enzyme können anhand der bekannten Enzymklassen zugewiesen werden.

Gegenstand der Erfindung ist daher ein Verfahren zur Herstellung von biogenen Stoffen, dadurch gekennzeichnet, dass
a) mindestens ein Ausgangsstoff mit
b) mindestens einem Enzym versetzt wird und das erhaltene Produkt aus b) mit
c) mindestens einer Leberzelle versetzt wird,
   oder
b') mindestens eine Leberzelle versetzt wird und das erhaltene Produkt aus b) mit
c') mindestens einem Enzym versetzt wird,
   und
d) mindestens ein biogener Stoff isoliert wird. (nach- und vorstehend "erfindungsgemäßes Verfahren")

Überraschender Weise lassen sich kostengünstig leberrelevante biogene Stoffe in hoher Ausbeute kontinuierlich oder diskontinuierlich herstellen, wobei vorteilhaft neue stereoselektive Verbindungen (Metaboliten) erhalten werden können.

Der Ausgangstoff enthält mindestens eine chemische Substanz, ein Substanzgemisch, insbesondere einen Arznei- oder Wirkstoff.

Weiterhin ist bevorzugt, dass das Enzym aus b) und c') im Gegensatz zu der Leberzelle aus einem anderen Organismus stammt.

In einer bevorzugten Ausführungsform wird ein erstes Enzym aus Pilzen, Hefen, Algen oder Bakterien erhalten und die eingesetzten Leberzellen sind humane Hepatozyten. Pilze sind jedoch erfindungsgemäß bevorzugt.

Die Enzyme können nach bekannten Verfahren aus den Organismen isoliert und aufgereinigt werden. Weiterhin können solche Enzyme rekombinant in einem Wirt hergestellt werden.

"Leberzelle" im Sinne dieser Erfindung bedeutet, dass die Zelle mindestens Enzyme zur Biotransformation aufweist, insbesondere Phase-I-Enzyme und/oder Phase-II-Enzyme und folglich hinreichend eine Leberfunktion ausführt. Die Leberzelle ist vorzugsweise eine solche, die aus humanen Leberzellen oder Säugerleberzelle erhalten werden kann. Solche Hepatozyten können z.B. aus den Lehren gemäß WO 2009/030217A2 und WO 2012/045731A1 bereitgestellt werden, umfassend genetisch veränderte Hepatozyten, welche z.B. ein Proliferationsgen aufweisen, so genannte proliferierende Leberzellen.

Ebenfalls sind erfindungsgemäß genetisch veränderte Leberzellen umfasst, die (transgen) beliebig verändert sein können. Beispielsweise können solche Leberzellen mittels viralen Vektoren (z.B. Lentiviren, z.B. DE 69830663 T2, EP 1175436 B1) hergestellt werden. Die Herstellung solcher genetisch veränderter Leberzellen ist dem Fachmann bekannt, vorzugsweise können Phase I und Phase II Enzyme ebenfalls rekombinant vorliegen. Weiterhin können Leberzelllinien erfindungsgemäß umfasst sein, die dem Fachmann bekannt sind, wie nicht abschließend käuflich erwerblich (www.cell-lines-service.de) Chang-Liver (Humane Leber Zell-Linie), Hep-G2 (Humane Hepatom Zell-Linie), HuH-7 (Humane Hepatoblastom Zell-Linie), PLC-PRF-5 (Humane Hepatom Zell-Linie), SK-HEP-1 (Humane Leber Adenokarzinom Zell-Linie) als auch z.B. Fa2N-4, Hep3B, BC2, HepaRG.

Zu den erfindungsgemäßen relevanten Phase I Enzymen zählen insbesondere Cytochrom P450 System, Alkohol Dehydrogenasen, Aldehyd Dehydrogenasen, Peroxidasen, Glutathionperoxidase, Esterasen, Hydrolasen.

Zu den erfindungsgemäßen relevanten Phase lI Enzymen zählen insbesondere Glucuronyltransferasen, Sulfotransferasen, Glutathion-S-Transferase, Methyltransferasen, Aminotransferasen / Transaminasen, Acetyltransferasen.

Die Verfahrensschritte b) (b') und c) (c') können in einer Ein-Topfvorrichtung durchgeführt werden. Eine solche Ein-Topfvorrichtung ist bevorzugt als Bioreaktor ausgestaltet.

In einer weiteren bevorzugten Ausführungsform sind die Verfahrensschritte b) (b') und c) (c') voneinander räumlich getrennt, z.B. mittels zwei oder mehreren voneinander getrennten Bioreaktoren. Dies kann ebenfalls in der Weise erfolgen, dass ein Bioreaktor zwei oder mehr Schichten aufweist, wobei jeweils in einer Schicht mindestens ein erfindungsgemäßer o.g. Verfahrensschritt durchgeführt wird.

Bevorzugt ist jedoch der Einsatz von zwei oder mehreren unabhängigen Bioreaktoren, die vorzugsweise miteinander gekoppelt sein können.

Daher betrifft die Erfindung ein erfindungsgemäßes Verfahren, wobei mindestens ein Bioreaktor, vorzugsweise zwei oder mehrere Bioreaktoren zur Ausführung der Verfahrensschritte b) (b') und c) (c') eingesetzt werden, und mindestens ein Schritt b) (b') und c) (c') auch wiederholt oder nachgeschaltet werden kann.

Die Bioreaktoren können mit Rührern und anderen üblichen Hilfsmitteln ausgestattet sein. Geeignete Bioreaktoren zur Kultivierung sind solche wie Hohlfaser-Bioreaktoren, Rührkesselreaktor (Fermenter), Wirbelschichtreaktor (Aggregate oder poröse Trägermaterialien), Festbettreaktoren mit Hohlfaser- bzw. Flachbettmembranen (Verfahren ist Tangentialflussfiltration), Plug-Flow-Reactor, Spinner Flaschen Kultivierung (siehe Horst Chmiel (Hrsg.) Bioprozesstechnik (2011), Spektrum Akademischer Verlag, Heidelberg).

In einer weiteren bevorzugten Ausführungsform sind die Bioreaktoren miteinader gekoppelt. Die Kopplung kann beispielsweise durch eine beliebige Verbindung zweier oder mehrerer Bioreaktoren erfolgen, z.B. entlang einem Flussgradienten, wobei Medium, Kulturflüssigkeit oder Überstand von einem Bioreaktor zu einem anderen Bioreaktor gelangt.

Zur Durchführung der Erfindung können die Enzyme gemäß b) und c') in der Weise eingesetzt werden, dass die Enzyme in einem Enzym-Membranreaktor oder immobilisiert auf einem beliebigen Träger vorliegen. Weiterhin geeignet sind Scaffolds, Mikrosomen oder zellfreie Systeme.

Geeignete Zellkulturen sind dem Fachmann einschlägig bekannt und käuflich erwerblich (siehe Beispiel).

Die Isolierung der erhaltenen biogenen Stoffe (Down-Stream-Prozess) oder Zwischenprodukte kann mittels Aufschließen der Leberzellen oder Aufreinigung aus dem Überstand erfolgen. Die erhaltenen Produkte und Verbinungen können z.B. mittels LC/MS identifziert werden.

Der Begriff "biogene Stoffe" im Sinne dieser Erfindung bedeutet, dass das erfindungsgemäße Verfahren die Herstellung von Stoffen und zwar vorzugsweise von neuen Stereoisomeren erlaubt.

Nachfolgende Beispiele und Figuren dienen zur näheren Erläuterung der Erfindung, ohne jedoch die Erfindung auf diese Beipiele und Figuren zu begrenzen.

### Beispiele und Figuren:

### Beispiel 1:

### Herstellung von 4'-Hydroxy-Diclofenac durch Leberzellen

HepG2 Zellen (ATCC HB-8065) werden in dem Medium DMEM PAA (Pasching, Austria) kultiviert, welches mit 10 % FCS Gold, 2 mM L-Glutamin, 100 U/ml Penicillin und 0,1mg/ml Streptomycin versetzt wird. Primäre humane Hepatozyten (pHHs) werden kommerziell von z.B. TebuBio (Le Perray-en-Yvelines, Frankreich) oder Promocell (Heidelberg, Deutschland) bezogen. Die Zellen werden auf 50 µg/ml Kollagen Typ I (Rattenschwanz, BD Biosciences, San Jose, USA) beschichtete Zellkulturplatten ausgesät und bei 37°C und 5 % CO2 in Hepatozyten Growth Medium (Promocell GmbH, Heidelberg, Deutschland) oder dem Fachmann bekanntem gleichwertigen Zellkulturmedien anderer Hersteller kultiviert. Diese pHHs können direkt für die Metabolitproduktion eingesetzt werden oder nach Erzeugung proliferationskompetenter Leberzellen. Dafür werden Proliferationsgene mit einem lentiviralen Vektor in die Zellen transduziert. Diese Technik ist in der WO2009/030217A2 (Braspenning et al. (2007)) beschrieben. Zellklone, welche die Proliferationsfähigkeit im Vergleich zu nicht infizierten primären Hepatozyten erhalten haben, wachsen zu in den Zellkulturschalen sichtbaren Kolonien aus und können über Trypsinierung abgelöst und isoliert werden. Die proliferienden Hepatozyten-Klone (Zelllinien) werden in Bezug auf ihre Leberzellmarker charakterisiert. Über quantitative real-time PCR (qRT-PCR) kann nachgewiesen werden, dass die für die Bildung von Phase-I- und Phase-II-Metaboliten notwendigen Enzyme exprimiert werden. Zur Definition von Sequenzen zur Generierung von Primern kann der Fachmann Datenbanken wie NCBI Genbank (http://www.ncbi.nlm.nih.gov/genbank) oder European Nucleotide Archive (http://www.ebi.ac.uk/ena/) verwenden. Für Phase I sind vor allem die Enzyme der Cytochrom- P450 (CYP) Familie relevant. Dazu gehören unter anderem die CYPs 1A2, 2B6, 2C6, 2C8, 2C9, 2C19, 3A4, 3A5, 3A6, 2D5, 2E1. Für Phase II sind u.a. die UDP-Glucuronosyltransfersasen (UGTs), Sulfotransferasen, (STs), N-Acetyltransferasen (NATs), Glutathion-S- Transferasen (GSTs), Methyltransferasen (z.B. TPMT, COMT etc.) relevant. Für alle diese Phase-I- und Phase-II-Enzyme lassen sich geeignete Primer zum Nachweis über qRT-PCR aus Datenbankeinträgen (NCBI, ENA) ableiten. Alternativ kann die Expression von relevanten Enzymen der Biotransformation über Immunfluoreszenz oder Western Blot mittels spezifischer Primärantikörper, welche an die exprimierten Phase-I- und Phase-II-Enzyme binden, nachweisen.

Eine geeignete Hepatozyten-Kultur, ob proliferationsfähige Hepatozyten (z.B. Zellklon HepaFH3) oder nicht proliferationsfähige pHHs werden dann zu z.B. 5x105 Zellen pro well in Kollagen I beschichtete 24-well- Platten ausgesät. Nach 2-7 Tagen in Kultur wird Medium mit 100 µM Diclofenac (Sigma-Aldrich, St. Louis, USA) zu den Zellen gegeben. Die Inkubation findet für 2-24 Stunden im Brutschrank bei 5 % CO2 und 37 °C in Hepatozytenmedium statt. Überstände dieser Zellkulturen werden für Spurenanalytik mit HPLC verwendet. Dazu kann das Gerät: VWR-Hitachi Elite LaChrom series HPLC mit Photodiode array L-2455 (Säule: Phenomenex Kinetex C18, 4.6x150 mm, 5 µm Partikelgröße, 100 Å; Vorsäule: RP18; 4,6x2 mm) verwendet werden. Der Nachweis des gebildeten Metabolits 4'-Hydroxy-Diclofenac kann auch über LC-MS mit einem Waters Alliance HPLC System (Säule: Phenomenex Luna C18[2] 2,0x150 mm, 5 µm Partikelgröße, 100 Å; Vorsäule: RP18; 4x3 mm in Kombination mit einem ZMD SingleQuad-MS Detektionsystem (ESI+; cone voltage: 30eV) durchgeführt werden.

Wie in der Tabelle 1 dargestellt, werden bei HepaFH3 (proliferationsfähige Leberzellen) sowie bei HepG2 (Leberzelllinie) sowie bei primären humanen Hepatozyten (pHH) verschiedener Donoren für das Zellsystem jeweils charakteristische Mengen von Metaboliten gefunden.

**Tabelle 1: Metabolismus von Diclofenac und Testosteron in verschiedenen Leberzellsystemen**

| Substanz | Metabolite | HepaFH3 (n = 6) | HepG2 (n = 3) | pHH Donor 1 | pHH Donor 2 |
|---|---|---|---|---|---|
| Diclofenac | 4'-OH-Diclofenac | 7,13 ± 2,22 | n.n. | 15,3 | 59,4 |
| Testosteron | Androstendion | 84,33 ± 11,59 | 35,32 ± 0,1 | 30,9 | 72, 2 |
| | OH-Testosteron | 3,02 ± 1,06 | 2,76 ± 0,1 | 6,1 | 23,0 |

Die über HPLC und LC-MS Analyse erhaltenen Werte sind in pmol/(min*106 Zellen); n.n. = keine Metabolite nachweisbar; pHH-primäre humane Hepatozyten

### Beispiel 2:

### Herstellung des Phase-II-Metaboliten 7-Hydroxyumarin-Glucuronid aus 7-Ethoxycumarin über ein zweistufiges Verfahren.

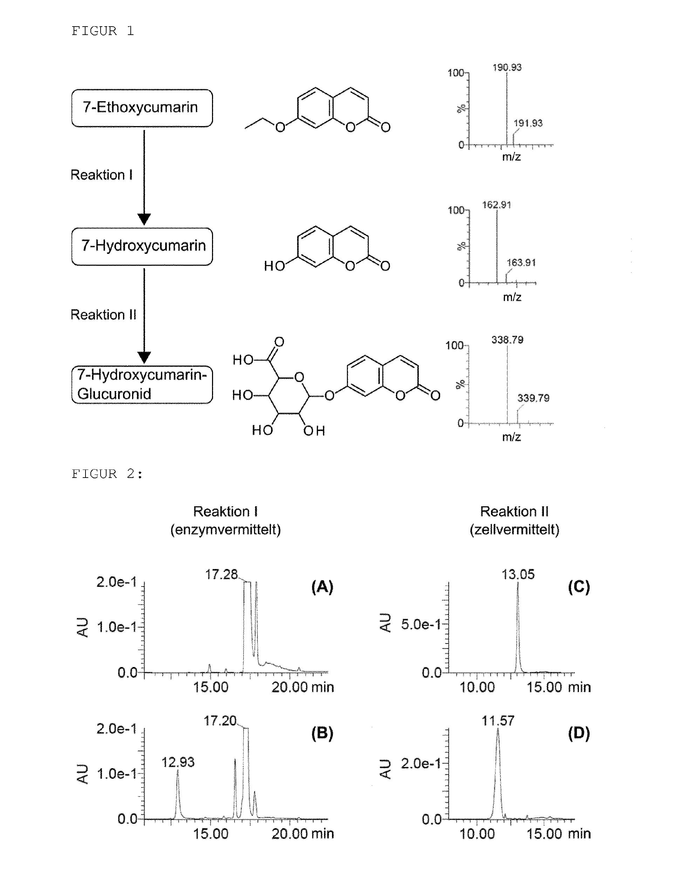

Bei diesem Ausführungsbeispiel wurde die Ausgangssubstanz 7-Ethoxycumarin in einer ersten Reaktion zum Phase-I-Metaboliten 7-Hydroxycumarin katalysiert durch pilzliche Peroxygenasen umgesetzt. In einer Folgereaktion wurde dieses Zwischenprodukt in einem Zell-Bioreaktor zum Phase-II-Metaboliten (Glucuronid) biotransformiert. Diese zweite Reaktion erfolgte an primären oder molekularbiologisch veränderten humanen Leberzellen. Mit diesem Beispiel wird die kombinierte Synthese für humane *First*-*pass*-Arzneimetabolite (Phase I und II) aus einer enzym-und zellvermittelten Biotransformation verdeutlicht ((Ullrich *et al.* (2007); Kinne et *al.* (2009); Poraj-Kobielska *et al.* (2011)). In Figur 2 ist das Prinzip der Reaktionsfolge dargestellt.
Für die Reaktion I wurde ein Reaktionsansatz von 0,5-200 ml verwendet (0,5-5 mM Substrat; Peroxygenase des Pilzes *Agrocybe aegerita* mit einer Aktivität von 4-400 Units; Phosphatpuffer 50 mM (pH7); 4 mM Ascorbinsäure). Nach Zugabe des Co-Substrat H₂O₂ (Aktivator der Reaktion; 5 mM) und einer Reaktionszeit von 60 min wurde das gebildete Produkt (7-Hydroxycumarin) *via* LC-MS bestimmt (λmax = 323 nm). In diesem Beispiel handelt es sich somit um eine O-Deethylierungsreaktion von 7-Ethoxycumarin zum Phase-I-Metaboliten 7-Hydroxycumarin. In der zweiten Reaktion werden Zellen (humane Hepatozyten) zur Biotransformation von 7-Hydroxycumarin zum Phase-II-Metaboliten 7-Hydroxycumarin-Glucuronid verwendet (Braspenning *et al.* (2007); Hansen *et al.* (2013); Burkard *et al.* (2012)). Die Hepatozyten wurden in einem Hohlfaser-Bioreaktor (CellFiber-System; Fläche: 75 cm²; Zellzahl: 1x10⁸ Zellen) für mindestens 7 Tage vorinkubiert. Anschließend erfolgte die Applikation des Substrates (100 µM) über einen Mediumswechsel. Die Umsetzung erfolgte im Hepatocyte Growth Medium (Promocell GmbH) über einen Zeitraum von 5-6 Tagen nahezu vollständig (Figur 2).
Die HPLC-Analytik der Substrate und deren Metabolite wurde an einem LC-MS-System (Waters alliance HPLC-System mit ZMD SingleQuad-MS (ESI+; cone voltage: 30eV) unter Verwendung einer RP-HPLC-Säule (Phenomenex Luna C18(2) 2x150mm, 100 Å, Vorsäule:RP18; 4x3mm) durchgeführt. Erforderliche Quantifizierungen wurden mittels Kalibriergeraden bei verbindungstypischen Absorptionsmaxima (Diclofenac: λmax = 270 nm; Propranolol: λmax = 220 nm) verwendet. Unter Verwendung der Laufmittel A (Wasser + 0,1 % Ameisensäure) und Laufmittel B (Acetonitril) setzte sich der lineare HPLC-Gradient wie folgt zusammen: 5 min (5 % LM B/95 % LM A), von 5 bis 20 min ändert sich das Mischungsverhältnis der Fließmittelzusammensetzung linear auf 100 % Laufmittel B. Die Flussrate betrug 0,5 ml/min (Hansen *et al.* (2013)).
**Figur 1****:** Dargestellt ist ein zweistufiges Verfahren zur Umsetzung von dem Substrat 7-Ethoxycumarin über ein Phase-I-Zwischenprodukt (7-Hydroxycumarin) zu einem Phase-II-Metaboliten (7-Hydroxycumarin-Glucuronid). Reaktion I (Modul pilzliche Peroxygenase): Grundlage sind extrazelluläre Enzyme pilzlichen Ursprungs (Peroxygenase; *Agrocybe aegerita* [1, 2]; Reaktion II (Modul Hepatozyten): In einer Folgereaktion wird der Phase-II-Metabolit (7-Hydroxycumarin-Glucuronid) mit einem präparativen 3D-Zellkulturverfahren (Hohlfasermodul-System, Fibercell Inc.) mit veränderten humanen Leberzellen (Hepatozyten) generiert.
**Figur 2****:** HPLC-Chromatogramme (λₘₐₓ=323nm): Umsetzung von 7-Ethoxycumarin (ret. time = 17,2 min) zu 7-Hydroxycumarin (ret. time = 12,9 min) mit fungaler Peroxygenase ((A) Reaktionszeit: 0 h; (B) Reaktionszeit: 0,5 h) in der ersten Reaktion. In der zweiten Folgereaktion ist die Biotransformation von 7-Hydroxycumarin (ret. time = 13,0 min) zum Phase-II-Metabolit 7-Hydroxycumarin-Glucuronid (ret. time = 11,6 min) mit humanen Hepatozyten dargestellt ((C) Reaktionszeit: 0 h; (D) Reaktionszeit: 24 h)

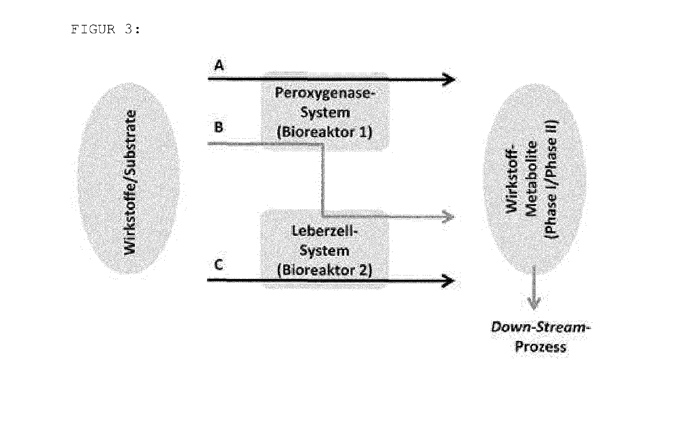

**Figur 3****:** Darstellung des Erfindungsprinzips.

### Referenzen

### Literatur

1. Elaut G, Henkens T, Papeleu P, Snykers S, Vinken M, Vanhaecke T, Rogiers V (2006): Molecular mechanisms underlying the dedifferentiation process of isolated hepatocytes and their cultures. Current drug metabolism 7 (6): 629-60.
2. Arimoto R (2006): Computational models for predicting interactions with cytochrome p450 enzyme. Current topics in medicinal chemistry 6 (15): 1609-18.
3. Shimada T, Yamazaki H, Mimura M, Inui Y, Guengerich FP (1994): Interindividual variations in human liver cytochrome P-450 enzymes involved in the oxidation of drugs, carcinogens and toxic chemicals: studies with liver microsomes of 30 Japanese and 30 Caucasians. The Journal of pharmacology and experimental therapeutics 270 (1): 414-23.
4. Lamb DC, Waterman MR, Kelly SL, Guengerich FP (2007): Cytochromes P450 and drug discovery. Current opinion in biotechnology 18 (6): 504-12.
5. Chang CY, Schiano TD (2007): Review article: drug hepatotoxicity. Alimentary pharmacology & therapeutics 25 (10): 1135-51.
6. Walker K, Ginsberg G, Hattis D, Johns DO, Guyton KZ, Sonawane B (2009): GENETIC POLYMORPHISM IN N-ACETYLTRANSFERASE (NAT): POPULATION DISTRIBUTION OF NAT1 AND NAT2 ACTIVITY. Journal of Toxicology and Environmental Health-Part B-Critical Reviews 12 (5-6): 440-472
7. Ullrich R, Kinne M, Hofrichter M, Scheibner K (2007): Verfahren zur O-Dealkylierung von Alkyarylethern. *Deutsches Patentamt* DE10 2007 058 741.6.
8. Kinne M, Poraj-Kobielska M, Aranda E, Ullrich R, Hammel KE, Scheibner K, Hofrichter M (2009): Regioselective preparation of 5-hydroxypropranolol and 4'-hydroxydiclofenac with a fungal peroxygenase. Bioorganic & medicinal chemistry letters 19 (11): 3085-7.
9. Poraj-Kobielska M, Kinne M, Ullrich R, Scheibner K, Kayser G, Hammel KE, Hofrichter M (2011): Preparation of human drug metabolites using fungal peroxygenases. Biochemical pharmacology 82 (7): 789-96.
10. Hansen M, Herzog N, Miethbauer S, Schmidtke KU, Lupp A, Sperling S, Seehofer D, Damm G, Scheibner K, Küpper J-H (2013): Primary-like human hepatocytes genetically engineered to obtain proliferation competence display hepatic differentiation characteristics in monolayer and organotypical spheroid cultures. Drug metabolism and disposition: the biological fate of chemicals (submitted).
11. Burkard A, Dahn C, Heinz S, Zutavern A, Sonntag-Buck V, Maltman D, Przyborski S, Hewitt NJ, Braspenning J (2012): Generation of proliferating human hepatocytes using Upcyte(R) technology: characterisation and applications in induction and cytotoxicity assays. Xenobiotica; the fate of foreign compounds in biological systems 42 (10): 939-56.

## Patentansprüche

1. Verfahren zur Herstellung von biogenen Stoffen, **dadurch gekennzeichnet, dass**
a) mindestens ein Ausgangsstoff mit
b) mindestens einem Enzym versetzt wird und das erhaltene Produkt aus b) mit
c) mindestens einer Leberzelle versetzt wird,
oder
b') mindestens einer Leberzelle versetzt wird und das erhaltene Produkt aus b) mit
c') mindestens einem Enzym versetzt wird,
und
d) mindestens ein biogener Stoff isoliert wird.

2. Verfahren nach Anspruch 1, **dadurch gekennzeichnet, dass** der Ausgangstoff mindestens eine Substanz enthält, insbesondere einen Arznei- oder Wirkstoff.

3. Verfahren nach Anspruch 1 oder 2, **dadurch gekennzeichnet, dass** das Enzym aus b) und c') im Gegensatz zu der Leberzelle aus einem anderen Organismus stammt.

4. Verfahren nach einem der vorhergehenden Ansprüche, wobei mindestens ein Bioreaktor, vorzugsweise zwei oder mehrere Bioreaktoren zur Ausführung der Verfahrensschritte b) (b') und c) (c') eingesetzt werden, und mindestens ein Schritt b) (b') und c) (c') auch wiederholt oder nachgeschaltet werden kann.

5. Verfahren nach einem der vorhergehenden Ansprüche, wobei das Enzym ausgewählt ist aus Oxidoreduktasen, insbesondere Monooxygenasen, Dioxygenasen, Oxidasen, Dehydrogenasen, Reduktasen und Peroxygenasen, Enzyme der Biotransformation, insbesondere Phase-I-Enzyme und/oder Phase-II-Enzyme, Esterasen, Hydrolasen und Transferasen.

6. Verfahren nach einem der vorhergehenden Ansprüche, wobei die Leberzelle ausgewählt ist aus einer Zelle enthaltend mindestens Enzyme zur Biotransformation, insbesondere Phase-I-Enzyme und/oder Phase-II-Enzyme, humane Leberzelle, Säugerleberzelle, genetisch veränderte Hepatozyten, proliferierenden Leberzellen, Leberzelllinien.

7. Verfahren nach einem der vorhergehenden Ansprüche, wobei Phase I Enzyme Cytochrom P450 System, Alkohol Dehydrogenasen, Aldehyd Dehydrogenasen, Peroxidasen, Glutathionperoxidase, Esterasen, Hydrolasen umfassen und/oder Phase lI Enzyme Glucuronyltransferasen, Sulfotransferasen, Glutathion-S-Transferase, Methyltransferasen, Aminotransferasen / Transaminasen, Acetyltransferasen umfassen.
